# EUROPÄISCHE PATENTANMELDUNG

(11) **EP 3 133 063 A1**
(43) Veröffentlichungstag der Anmeldung: **22.02.2017**
(21) Anmeldenummer: 15181535.4
(22) Anmeldetag: 19.08.2015
(51) Int. Cl.: C07D 271/113

(54) **VERFAHREN ZUR HERSTELLUNG VON 2-AMINO-1,3,4-OXADIAZOLEN**

(71) Anmelder: Bayer CropScience AG, 40789 Monheim am Rhein (DE)
(72) Erfinder: FUNKE, Christian, 42799 Leichlingen (DE); PAZENOK; Sergii, 42699 Solingen (DE); LUI, Norbert, 51519 Odenthal (DE)
(74) Vertreter: BIP Patents

(57) **Zusammenfassung**

Es wird ein Verfahren zur Herstellung von 2-Amino-1,3,4-oxadiazolen der Formel (I) beschrieben. 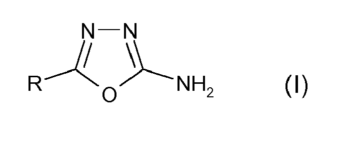 worin R für Wasserstoff, Alkyl, Haloalkyl oder Aryl steht.

## Beschreibung

Die Erfindung betrifft ein Verfahren zur Herstellung von 2-Amino-1,3,4-oxadiazolen sowie deren Verwendung als Zwischenprodukte für die Synthese von Feinchemikalien und von agrochemischen Wirkstoffen.

2-Amino-1,3,4-oxadiazole der Formel (I) stellen ein wichtiges Strukturelement bei einer Vielzahl von agronomisch wirksamen Substanzen dar, wie sie z.B in WO 2012/126932 A1 und WO 2013/087577 A1 offenbart werden.

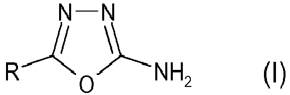

2-Amino-1,3,4-oxadiazole können z. B. aus Carbonsäure-Hydraziden und Trimethylsilylthiacyanat in Gegenwart von elementarem Iod hergestellt werden. Diese Methode ist beispielsweise aus RSC Advance, 2013, 3, 7684 bekannt. In J.Heteroc.Chem. 1972, 9, 153 wird die Synthese von 5-Alkyl- und 5-Aryl-2-amino-1,3,4-oxadiazolen ausgehend von Carbonsäure-Hydraziden und Bromcyan beschrieben. Die in diesen Schriften genannten Verfahren sind jedoch auf eine Durchführung im Labormaßstab beschränkt, da sie eine Reihe von Nachteilen aufweisen und somit nicht für eine industrielle Produktion anwendbar sind: Teure Ausgangsstoffe, mangelnde Lagerstabilität der Ausgangsstoffe, unzureichende Ausbeuten und grosse Abfallmengen.

Aufgabe der vorliegenden Erfindung ist die Bereitstellung eines Verfahrens zur Herstellung von 2-Amino-1,3,4-oxadiazolen, welches die Nachteile der aus dem Stand der Technik bekannten Verfahren überwindet.

Es wurde nun gefunden, dass 2-Amino-1,3,4-oxadiazole kostengünstig und in hohen Ausbeuten hergestellt werden können durch Reaktion von Carbonsäure-Hydraziden und Chlorcyan. Wesentliche Vorteile dieses Verfahrens bestehen in der guten Verfügbarkeit der Ausgangsstoffe und der hohen Ausbeute.

Ein Gegenstand vorliegender Erfindung ist somit ein Verfahren zur Herstellung von 2-Amino-1,3,4-oxadiazolen der allgemeinen Formel (I), dadurch gekennzeichnet, dass Carbonsäure-Hydrazide der Formel (II) mit Chlorcyan bei einer Temperatur von -5 bis 40°C in Gegenwart eines Lösungsmittels umgesetzt werden, und worin der Substituent R wie nachfolgend definiert ist: 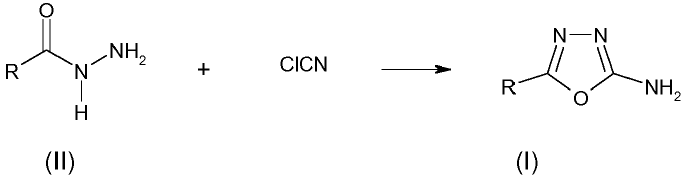 R bedeutet Wasserstoff, (C₁-C₈)-Alkyl, Halogen-(C₁-C₈)-Alkyl, (C₁-C₄)-Alkoxy-und (C₁-C₆)-alkyl oder durch p Reste aus der Gruppe bestehend aus Fluor, Chlor, Brom, Iod, (C₁-C₄)-Alkyl, (C₁-C₄)-Alkoxy und (C₁-C₄)-Alkylthio substituiertes Phenyl, 1-Naphthyl, 2-Naphthyl oder Pyridyl,
p bedeutet 0, 1, 2, 3 oder 4.

Die Carbonsäure-Hydrazid der Formel (II) sind kommerziell erhältlich oder nach einfachen dem Fachmann bekannten Methoden herstellbar. Chlorcyan ist kommerziell erhältlich.

In den Formeln (I) und (II) können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl.

Halogen bedeutet Fluor, Chlor Brom oder Iod, bevorzugt Fluor oder Chlor.

Ist eine Gruppe mehrfach durch Reste substituiert, so ist darunter zu verstehen, daß diese Gruppe durch ein oder mehrere gleiche oder verschiedene der genannten Reste substituiert ist.

In dem erfindungsgemäßen Verfahren bedeutet der Substituent R vorzugsweise Wasserstoff, Methyl, Ethyl, Propyl, iso-Propyl, Butyl, Pentyl, Difluormethyl, Trifluormethyl, Pentafluorethyl oder Phenyl.

In einer besonders bevorzugten Ausführungsform steht R für Wasserstoff, Methyl, Ethyl oder Phenyl.

Das erfindungsgemäße Verfahren wird in der Regel so durchgeführt, daß das Carbonsäure-Hydrazid der Formel (II) mit 1 bis 3 Äquivalenten Chlorcyan, vorzugsweise 1 bis 1,5 Äquivalente, bei einer Temperatur von -5 bis 40°C, vorzugsweise 0 bis 25°C in Gegenwart eines Lösungsmittels umgesetzt werden.

In Abhängigkeit von der Art des Substituenten R kann es zweckmäßig sein das erfindungsgemäße Verfahren in Gegenwart von 0,5 bis 3 Äquivalenten einer Base, vorzugsweise 0,5 bis 1,5 Äquivalente, bezogen auf das Carbonsäure-Hydrazid der Formel (II), durchzuführen.

Das erfindungsgemäße Verfahren kann in Abhängigkeit von der Art des Substituenten R in einer Vielzahl von Lösungsmitteln oder in Mischungen von ihnen durchgeführt werden. Geeignete Lösungsmittel sind Wasser, Alkohole wie Methanol, Ethanol und iso-Propanol; aliphatische, alicyclische oder aromatische Kohlenwasserstoffe wie Petroleether, n-Hexan, n-Heptan, Cyclohexan, Methylcyclohexan, Toluol, Xylol und Dekalin; halogenierte Kohlenwasserstoffe wie Chlorbenzol, Dichlorbenzol, Dichlormethan, Chloroform, Tetrachloromethan, Dichloroethan und Trichloroethan; Ether wie Diethylether, Diisopropylether, Methyl-tert-butylether, Methyl-tert-amylether, Dioxane, Tetrahydrofuran, 1,2-Dimethoxyethan, 1,2-Diethoxyethan und Anisol; Nitrile wie Acetonitril, Propionitril, n-Butyronitril, iso-Butyronitril und Benzonitril; Amide wie N,N-Dimethylformamid, N,N-Dimethylacetamid, N-Methylformanilid, N-Methylpyrrolidon und Hexamethylphosphoramid; Sulfoxide wie Dimethylsulfoxid sowie Sulfone wie Sulfolan, Aceton oder Mischungen dieser Lösungsmittel.

Bevorzugte Lösungsmittel sind Tetrahydrofuran, Acetonitril, Methanol, Ethanol, Aceton, Wasser und deren Mischungen.

Geeignete Basen sind anorganische Basen wie Li₂CO₃, Cs₂CO₃, NaHCO₃, Na₂CO₃, KHCO₃, K₂CO₃, NaOH, NH₄OH, KOH, Ca(OH)₂,CaCO₃, Natriumacetat, sowie organische Basen wie Pyridin, Dimethylpyridin, Methylpyridin,Trimethylamin, Triethylamin, Tributylamin, Diazabicycloundecen und Triethylendiamin. Bevorzugt sind NaHCO₃, Na₂CO₃, KHCO₃ und K₂CO₃.

Die Verbindungen der Formel (II) und Chlorcyan haben in der Regel in dem erfindungsgemäßen Verfahren nach 1 bis 3 Stunden vollständig abreagiert.

Die Aufarbeitung und Isolierung der Verbindung der Formel (I) erfolgt dann auf üblichen und dem Fachmann bekannten Wegen. Üblicherweise wird das Lösungsmittel im Vakuum teilweise oder völlstandig entfernt und das Produkt aus dem Reaktionsgemisch durch einfache Filtration isoliert und, falls erforderlich, durch Umkristallieren weiter gereinigt.

Die nachfolgenden Herstellbeispiele erläuteren die Erfindung näher.

### Beispiel 1:

### Herstellung von 2-Amino-5-methyl-1,3,4-oxadiazol

In eine Lösung von 20.0 g (0.27 mol) Acethydrazid in 200 ml Methanol wurden bei 5°C langsam 17.2 g (0.28 mol) Chlorcyan eingeleitet. Die Reaktionslösung wurde auf 30°C erwärmt und 1 Stunde bei 30°C nachgerührt und dann auf 0°C abgekühlt. 19.5 g (0.14 mol) fein gemörsertes K₂CO₃ wurden in 5 Portionen so langsam zugegeben, daß die Temperatur nicht über 10°C stieg. Die resultierende Suspension wurde noch 3 Stunden bei Raumtemperatur nachgerührt und danach filtriert. Das Filtrat wurde eingeengt und der Rückstand aus 20 ml Wasser umkristallisiert. Man erhielt 22 g (81 % d. Theorie) des Produktes mit einem Festpunkt von 184-186°C.
NMR ¹H (DMSO-d⁶): 2.25 (s, 3H); 6.8 (s, 2H) ppm

### Beispiel 2:

### Herstellung von 2-Amino-5-phenyl-1,3,4-oxadiazol

In eine Lösung von 20.4 g (0.15 mol) Benzoylhydrazid in 200 ml Methanol wurden bei 5°C langsam 12.2 g (0.2 mol) Chlorcyan eingeleitet. Die Reaktionslösung wurde auf 30°C erwärmt und 1 Stunde bei 30°C nachgerührt und dann auf 0°C abgekühlt. 28 g (0.2 mol) fein gemörsertes K₂CO₃ wurden in 5 Portionen so langsam zugegeben, daß die Temperatur nicht über 10°C stieg. Die resultierende Suspension wurde noch 3 Stunden bei Raumtemperatur nachgerührt auf 20°C erhitzt und danach filtriert. Das Filtrat wurde eingeengt und der Rückstand aus Methanol umkristallisiert. Man erhielt 20.5 g (85 % d. Theorie) des Produktes mit einem Festpunkt von 250-252°C.

### Beispiel 3:

### Herstellung von 2-Amino-1,3,4-oxadiazol

Die analog der Methode in Beispiel 1 mit Formylhydrazid an Stelle von Acethydrazid durchgeführte Reaktion liefert nach Umkristallisation aus Aceton 2-Amino-1,3,4-oxadiazol in einer Ausbeute von 87 % und mit einem Festpunkt von 152-153°C.

## Patentansprüche

1. Verfahren zur Herstellung von 2-Amino-1,3,4-oxadiazolen der allgemeinen Formel (I), **dadurch gekennzeichnet, dass** Carbonsäure-Hydrazide der Formel (II) mit Chlorcyan bei einer Temperatur von -5 bis 40°C in Gegenwart eines Lösungsmittels umgesetzt werden, und worin der Substituent R wie nachfolgend definiert ist: 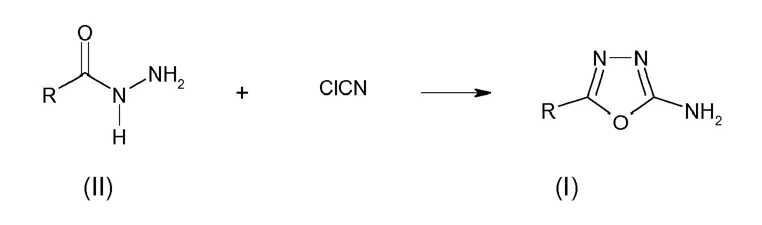 R bedeutet Wasserstoff, (C₁-C₈)-Alkyl, Halogen-(C₁-C₈)-Alkyl, (C₁-C₄)-Alkoxy-und (C₁-C₆)-alkyl oder durch Reste aus der Gruppe bestehend aus Fluor, Chlor, Brom, Iod, (C₁-C₄)-Alkyl, (C₁-C₄)-Alkoxy und (C₁-C₄)-Alkylthio substituiertes Phenyl, 1-Naphthyl, 2-Naphthyl oder Pyridyl,
p bedeutet 0, 1, 2, 3 oder 4.

2. Verfahren nach Anspruch 1, worin R für Wasserstoff, Methyl, Ethyl, Propyl, iso-Propyl, Butyl, Pentyl, Difluormethyl, Trifluormethyl, Pentafluorethyl oder Phenyl steht.

3. Verfahren nach Anspruch 1 oder 2, worin R für Wasserstoff, Methyl, Ethyl oder Phenyl steht.

4. Verfahren nach einem der Ansprüche 1 bis 3, wobei die Reaktion bei einer Temperatur von 0°C bis 25°C durchgeführt wird.

5. Verfahren nach einem der Ansprüche 1 bis 4, wobei 1 bis 3 Äquivalente Chlorcyan eingesetzt werden.

6. Verfahren nach einem der Ansprüche 5, wobei 1 bis 1,5 Äquivalente Chlorcyan eingesetzt werden.

7. Verfahren nach einem der Ansprüche 1 bis 6, wobei die Reaktion in Gegenwart von 0,5 bis 3 Äquivalenten einer Base, bezogen auf das Carbonsäure-Hydrazid der Formel (II), durchgeführt wird.

8. Verfahren nach Anspruch 7, wobei die Reaktion in Gegenwart von 0,5 bis 1,5 Äquivalenten einer Base durchgeführt wird.

9. Verfahren nach einem der Ansprüche 1 bis 8, wobei als Base Natriumhydrogencarbonat, Kaliumhydrogencarbonat, Natriumcarbonat oder Kaliumcarbonat verwendet wird.
